# EUROPÄISCHE PATENTANMELDUNG

(11) **EP 1 331 046 A2**
(43) Veröffentlichungstag der Anmeldung: **30.07.2003**
(21) Anmeldenummer: 03000797.5
(22) Anmeldetag: 14.01.2003
(51) Int. Cl.: B08B 15/02, B08B 9/027

(54) **Dekontaminierbare Sicherheitswerkbank**

(30) Priorität: 28.01.2002 DE 10203234
(71) Anmelder: KENDRO Laboratory Products GmbH, 63450 Hanau (DE)
(72) Erfinder: Glück, Walter, 63594 Hasselroth (DE); Ross, Gerd, 60388 Frankfurt (DE)
(74) Vertreter: Tomerius, Isabel, Dr.

(57) **Zusammenfassung**

Die Erfindung betrifft eine Sicherheitswerkbank (1) mit einem von einem Gehäuse (3) umschlossenen Arbeitsraum (2) und einem Lüftungssystems zur Belüftung dieses Arbeitsraums. Die Sicherheitswerkbank (1) umfasst weiterhin einen Ozongenerator (4), der Ozon in das Lüftungssystem einspeist, um die Sicherheitswerkbank zu dekontaminieren.
Die Erfindung betrifft weiterhin ein Verfahren zur Dekontamination einer Sicherheitswerkbank sowie die Verwendung für Ozon zu diesem Zweck.

## Beschreibung

Die Erfindung betrifft eine Sicherheitswerkbank, welche unter Verwendung von Ozon dekontaminiert werden kann.

Mikrobiologische Sicherheitswerkbänke der Klassen 1 bis 3 werden zur Bearbeitung biologischer Materialien im Laborbetrieb eingesetzt. Derartige Sicherheitswerkbänke verfügen über ein spezielles Lüftungssystem, welches verhindert, dass die biologischen Materialien bei ihrer Bearbeitung aus dem Arbeitsraum der Sicherheitswerkbank in die Umgebung austreten und den Bearbeiter kontaminieren. Die Sicherheitswerkbänke, vor allem diejenigen der Klasse 2, tragen außerdem dazu bei, Verunreinigungen des bearbeiteten Materials zu verhindern.

Bei der Bearbeitung biologischer Proben werden Aerosole der unter Umständen gefährlichen Agenzien innerhalb der Werkbank freigesetzt. Aufgrund der Luftzirkulation innerhalb der Sicherheitswerkbank werden die Aerosole innerhalb des gesamten Lüftungssystems der Geräts verteilt. Spezielle Hochleistungs-Schwebstofffilter (z. B. die sogenannten "HEPA"- und "ULPA"-Filter) verhindern, dass die biologischen Materialien aus der Sicherheitswerkbank nach außen gelangen und die Umgebung kontaminieren.

Wie bereits erwähnt, verteilen sich die Aerosole jedoch innerhalb der Sicherheitswerkbank. Um zu verhindern, dass neue Kulturen mit biologischen Fremdmaterialien verunreinigt werden, ist es daher erforderlich, das Innere der Sicherheitswerkbank regelmäßig, üblicherweise täglich, zu desinfizieren. Dies geschieht routinemäßig mit einem Sprühdesinfektionsmittel, welches auf die den Arbeitsraum umgebenden Flächen aufgesprüht und anschließend abgewischt wird. Auf diese Weise kann zwar der Arbeitsraum der Sicherheitswerkbank desinfiziert werden, nicht unmittelbar zugängliche Flächen wie Lüftungskanäle und Filter sind dagegen so nicht desinfizierbar. Dies gilt auch für die alternative Dekontaminationsmethode, welche regelmäßig bei Sicherheitswerkbänken angewendet wird, nämlich diejenige mit UV-C-Strahlen.

Um auf Dauer ein sicheres Arbeiten zu gewährleisten und das Einschleppen von Fremdmaterialien in die bearbeiteten Kulturen zu verhindern, müssen auch Lüftungskanäle und Filter regelmäßig dekontaminiert werden. International anerkannter Standard für die Totaldekontamination von Sicherheitswerkbänken ist die Begasung mit Formaldehyd-Dampf. Nachteil dieses Verfahrens ist jedoch, dass der Formaldehyd nach der Desinfektion mehrere Tage lang in der Sicherheitswerkbank verbleibt und so neben den abzutötenden Keimen auch die anschließend bearbeiteten Nutzkulturen innerhalb des Gerätes schädigt und zu einer Verfälschung der Untersuchungsergebnisse führt.

Darüber hinaus darf die Desinfektion mit Formaldehyd nur von eigens ausgebildetem Fachpersonal durchgeführt werden, welches sich wiederholten Prüfungen seiner Qualifikation unterziehen muss. Vor der Durchführung einer Desinfektion mit Formaldehyd ist außerdem die zuständige Behörde zu informieren. Der zeitliche und finanzielle Aufwand bei Durchführung der Formaldehyd-Desinfektion ist daher erheblich und für viele Unternehmen nicht mehr tragbar. Alternativ zur Formaldehyd-Desinfektion findet auch die Dekontaminierung mit Wasserstoffperoxid Anwendung. Dieses Desinfektionsmittel ist jedoch nur bei speziellen Agenzien wirksam und erfordert zudem sehr aufwendige und teure Spezialgerätschaften. Als Routinemaßnahme ist die Wasserstoffperoxid-Desinfektion daher nicht einsetzbar.

**Aufgabe** der Erfindung ist es entsprechend, eine Möglichkeit zur Desinfektion von Sicherheitswerkbänken anzugeben, welche routinemäßig mit möglichst geringem Aufwand zur Totaldekontaminierung von Sicherheitswerkbänken eingesetzt werden kann. Die Dekontamination sollte von nicht speziell ausgebildetem Personal durchführbar sein, ein breites Wirkungsspektrum zeigen und gleichzeitig eine Schädigung der Umgebung außerhalb der Sicherheitswerkbank praktisch ausschließen. Das Desinfektionsverfahren sollte zudem bei entsprechender Nachrüstung bei bereits existierenden Sicherheitswerkbänken anzuwenden sein.

Die Lösung dieser Aufgabe gelingt mit der Sicherheitswerkbank gemäß Anspruch 1 und dem Verfahren gemäß Anspruch 11. Bevorzugte Ausführungsformen und Verfahrensvarianten sind den jeweiligen Unteransprüchen zu entnehmen. Die Erfindung betrifft weiterhin die Verwendung von Ozon zur Dekontamination von Sicherheitswerkbänken.

Die Erfindung beruht also auf der Erkenntnis, dass sich Ozon zur Dekontaminierung von Sicherheitswerkbänken ausgezeichnet eignet. Ozon ist gasförmig, so dass auch die Dekontamination von Lüftungsschächten und Filtern mit diesem Desinfektionsmittel kein Problem darstellt. Ozon ist zudem ein anerkanntes Desinfektionsmittel, welches ein sehr breites Wirkungsspektrum aufweist. Die üblicherweise in Sicherheitswerkbänken auftretenden Verunreinigungen können durch Ozon beseitigt werden. Ozon weist zudem den Vorteil auf, dass es leicht erzeugbar ist und ebenfalls leicht wieder abgebaut werden kann. Als Abbauprodukt des Ozons entsteht Sauerstoff, welches für die Umgebung vollkommen unschädlich ist.

Die erfindungsgemäße Sicherheitswerkbank weist zur Erzeugung des Ozons einen Ozongenerator auf. Dieser ist so in der Sicherheitswerkbank angeordnet, dass das erzeugte Ozon in das Lüftungssystem der Sicherheitswerkbank eingespeist wird. Das Ozon wird mit der Lüftungsluft, welche für eine Reinluftatmosphäre innerhalb der Sicherheitswerkbank sorgt, durch alle Bereiche der Sicherheitswerkbank transportiert, in welche auch die aus den biologischen Materialien entstandenen Aerosole gelangen. Während der Dekontamination erreicht das Ozon also alle Bereiche des Arbeitsraums, des Lüftungssystems und der Filter, in welchen sich auch biologisches Material niedergeschlagen haben könnte.

Zur Erzeugung des Ozons kann jeder bekannte Ozongenerator verwendet werden. Besonders geeignet ist die Erzeugung des Ozons mittels UV-Strahlung oder auf elektrischem Wege über eine Funkenstrecke. Der Ozongenerator kann an jeder beliebigen Stelle der Sicherheitswerkbank angeordnet sein, welche eine Einspeisung des Ozons in den Lüftungsstrom der Sicherheitswerkbank erlaubt. Bevorzugt erfolgt die Anordnung des Ozongenerators außerhalb des Arbeitsraums der Sicherheitswerkbank, beispielsweise im Bereich eines Lüftungskanals des Lüftungssystems und vorzugsweise an einer leicht zugänglichen Stelle der Vorrichtung. Die Nachrüstung bereits vorhandener Sicherheitswerkbänke durch nachträglichen Einbau des Ozongenerators ist ohne Weiteres möglich.

Um zu verhindern, dass Ozon aus der Sicherheitswerkbank heraus nach außen gelangt, wird die Sicherheitswerkbank während der Dekontamination aerosoldicht verschlossen. Derartige dicht schließende Sicherheitswerkbänke sind grundsätzlich bereits bekannt und z. B. in der DE 4441784 C2 der Anmelderin beschrieben. Die Erfindung ist jedoch auch bei an sich nicht dicht schließenden Sicherheitswerkbänken anwendbar. Sie werden dann zweckmäßig vor der Dekontamination mit geeigneten Dichtmitteln gegen die Umgebung abgedichtet.

Um ein Austreten des Ozons nach Abschluss der Dekontamination zu vermeiden, weist die erfindungsgemäße Sicherheitswerkbank vorzugsweise eine Vorrichtung zum Abbau des Ozons auf. Diese ist zweckmäßig in die Abluftvorrichtung der Sicherheitswerkbank integriert. Bei der Abbauvorrichtung für das Ozon kann es sich beispielsweise um einen Filter oder einen Katalysator handeln. Geeignet ist zum Beispiel ein Platin-Iridium-Katalysator, welcher das Ozon zu Sauerstoff abbaut. Für den Fall, dass die Abbauvorrichtung ausgetauscht oder regeneriert werden muss, ist sie zweckmäßig so in der Sicherheitswerkbank angeordnet, dass sie leicht erreichbar ist.

Um zu verhindern, dass sich während des Ablassens des Dekontaminationsstroms aus der Sicherheitswerkbank im Inneren derselben ein Unterdruck bildet, ist in der Vorrichtung zweckmäßig ein Ventil vorgesehen, mit welchem Umgebungsluft zum Druckausgleich in das Belüftungssystem geführt werden kann. Das Ventil kann ebenfalls dazu dienen, dem Inneren der Werkbank Frischluft zuzuführen, damit stets genug Sauerstoff zur Erzeugung des Ozons zur Verfügung steht.

Um eine vollständige Dekontaminierung der erfindungsgemäßen Sicherheitswerkbank durch das Ozon zu gewährleisten, erfolgt die Dekontamination vorzugsweise über mehrere Stunden, besonders bevorzugt über Nacht. Das erfindungsgemäße Dekontaminationsverfahren ist so einfach anwendbar, dass es täglich durchgeführt werden kann. In einer besonders bevorzugten Variante der Erfindung weist die Sicherheitswerkbank eine Steuervorrichtung auf, mit welcher das Dekontaminationsverfahren zumindest teilweise automatisch durchgeführt werden kann. Bevorzugt erfolgt es vollautomatisch.

Zweckmäßig weist die Steuervorrichtung zudem Mittel zum Feststellen und Auswerten bestimmter Geräteparameter und/oder Mittel zum Anzeigen von Störungen auf. Beispielsweise kann die Steuerung so eingestellt sein, dass ein Starten der Ozonerzeugung erst dann möglich ist, wenn sichergestellt ist, dass die Sicherheitswerkbank gegenüber der Umgebung aerosoldicht verschlossen ist. Hierfür können beispielsweise im Bereich einer aerosoldicht verschließbaren Frontscheibe, welche die Arbeitsöffnung des Arbeitsraums verschließt, Sensoren vorhanden sein, welche an die Steuervorrichtung ein Signal senden, wenn die Frontscheibe tatsächlich dicht verschlossen ist. Erst wenn dieses Signal die Steuervorrichtung erreicht hat, wird der Ozongenerator durch die Steuervorrichtung gestartet. Weitere Geräteparameter, welche abgefragt werden können, sind z.B. das Funktionieren der Lüftung oder der ordnungsgemäße Anschluss und das Funktionieren der Abbauvorrichtung für das Ozon. Auch die Dauer der Ozonerzeugung kann einstellbar und automatisch steuerbar sein. Darüber hinaus kann vorgesehen werden, dass die Ozonerzeugung abgebrochen wird, wenn im Verlauf der Dekontamination eine Störung im System festgestellt wird. Zweckmäßig ist ebenfalls, die Frontscheibe während der Dekontamination automatisch zu verriegeln, um so ein unbeabsichtigtes Öffnen im Verlauf des Dekontaminationsverfahrens zu verhindern. Eine Notentriegelung kann ergänzend vorgesehen sein.

Die Anzeige von eventuellen Störungen kann beispielsweise auf optischem oder akustischem Wege geschehen. Für die optische Anzeige kann an der Sicherheitswerkbank beispielsweise eine Warnblinkleuchte oder ein Display angeordnet sein. Die akustische Warnung kann durch eine Sirene oder ähnliches abgegeben werden.

Die Erfindung wird nachfolgend anhand einer Zeichnung näher erläutert werden.

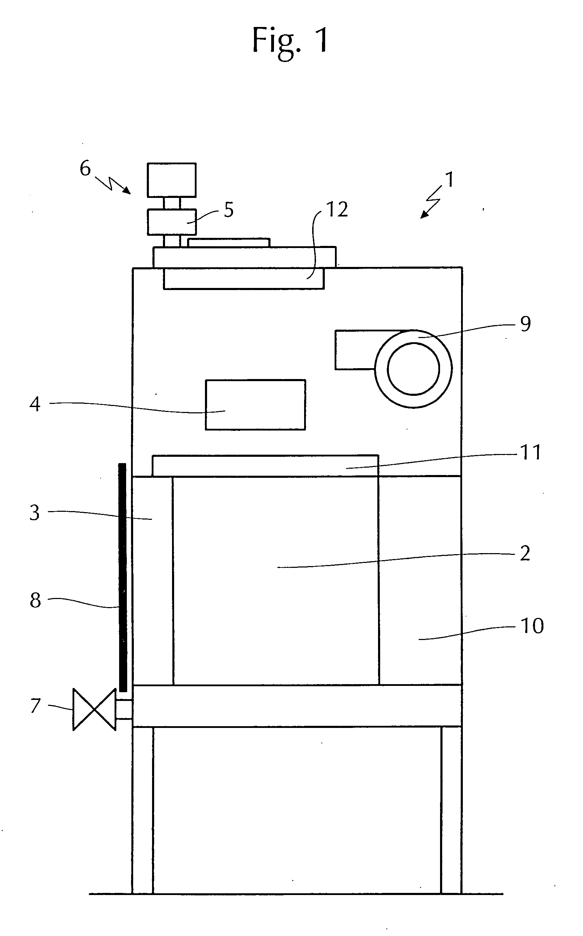

Figur 1 zeigt schematisch eine erfindungsgemäße Sicherheitswerkbank im Querschnitt.

Die in Figur 1 dargestellte Sicherheitswerkbank 1 entspricht im Wesentlichen einer herkömmlichen Sicherheitswerkbank, wie sie beispielsweise in der DE 4441784 C2 beschrieben ist. In einem Gehäuse 3 befindet sich ein Arbeitsraum 2, in welchem Proben bearbeitet werden können. Auf der Frontseite des Arbeitsraums 2 (in der Figur auf der linken Seite) ist der Arbeitsraum über eine Arbeitsöffnung zugänglich, welche mit einer höhenverstellbaren Frontscheibe 8 verschließbar ist. Während der Bearbeitung der Proben im Arbeitsraum 2 ist die Frontscheibe 8 zumindest teilweise nach oben geschoben. Um eine Verunreinigung der Proben im Arbeitsraum 2 und ein Austreten von Probenmaterial aus dem Arbeitsraum 2 nach außen zu verhindern, wird innerhalb der Sicherheitswerkbank 1 Luft zirkuliert. Hierfür ist im Raum oberhalb des Arbeitsraums 2 ein Lüfter 9 angeordnet. Zur Reinigung der zirkulierten Luft sind außerdem Filter 11 und 12 vorhanden.

Während der Bearbeitung der Proben verteilt sich Probenmaterial in Form von Aerosolen mit der zirkulierenden Luft im Innenraum der Sicherheitswerkbank 1. Auf diese Weise kann sich Probenmaterial an den Wänden des Arbeitsraums 2, im Lüftungskanal 10, im Lüfter 9 und in dem diesen umgebenden Lüftungsraum sowie in den Filtern 11 und 12 und außerdem in der Abluftvorrichtung 6 ablagern. Während die Wände des Arbeitsraums 2 sich mit herkömmlichen Sprühdekontaminationsmitteln dekontaminieren lassen, ist dieses Verfahren bei den übrigen genannten Räumen nicht möglich. Erfindungsgemäß gelingt die Dekontaminierung all dieser Räume jedoch durch gasförmiges Ozon, welches durch das Lüftungssystem der Sicherheitswerkbank zirkuliert wird. Vor und nach der Ozondekontaminierung kann der Arbeitsraum 2 zudem zusätzlich mit einem herkömmlichen Desinfektionsmittel gereinigt werden.

Zur Erzeugung des Ozons ist in der Sicherheitswerkbank 1 ein Ozongenerator 4 vorgesehen. Im gezeigten Fall befindet sich der Ozongenerator 4 im Lüftungsraum oberhalb des Arbeitsraums 2. In diesen Raum freigesetztes Ozon wird mit dem normalen Belüftungssystem mittels des Lüfters 9 durch den Innenraum der Sicherheitswerkbank 1 zirkuliert. Die mit Ozon angereicherte Lüftungsluft tritt also zunächst durch den Filter 11 in den Arbeitsraum 2 und von dort durch den Lüftungskanal 10 in den oberen Lüftungsraum zurück. Von hier gelangt Ozon auch in den Filter 12. Innerhalb dieses Kreislaufs wird die mit Ozon angereicherte Belüftungsluft mehrere Stunden lang zirkuliert. Die Einwirkzeit kann entsprechend den zuvor in Arbeitsraum 2 bearbeiteten Keimen gewählt werden.

Damit kein Ozon nach außen aus der Sicherheitswerkbank 1 austreten kann, wird diese vor Beginn der Ozongenerierung aerosoldicht verschlossen. Bei Sicherheitswerkbänken mit hermetisch schließenden Frontscheiben, wie sie beispielsweise in der DE 4441784 C2 beschrieben sind, geschieht dies einfach durch Schließen der Frontscheibe. In anderen Fällen wird die Sicherheitswerkbank mit geeigneten zusätzlichen Dichtmitteln verschlossen. Auch die Auslassvorrichtung 6 bleibt während der Ozonerzeugung geschlossen, so dass auch hier kein Ozon nach außen treten kann.

Nach Beendigung der Ozonbegasung wird die Begasungsluft durch die Abluftvorrichtung 6 aus der Sicherheitswerkbank nach außen entlassen. Hierfür wird der Auslass geöffnet, und gleichzeitig muss dafür gesorgt werden, dass entsprechend der abgelassenen Luft Umgebungsluft in das Lüftungssystem der Sicherheitswerkbank von außen eindringen kann. Dies ist entweder dadurch möglich, dass manuell eine Luftzufuhröffnung geschaffen wird, beispielsweise durch Öffnen der Frontscheibe. Bevorzugt ist es jedoch, an der Sicherheitswerkbank ein Ventil 7 vorzusehen, welches die Luftzufuhr in das Belüftungssystem sicherstellt. Über dieses Ventil kann dem Belüftungssystem zudem während der Ozonbegasung Luft zugeführt werden, damit im System hinreichend Sauerstoff als Ausgangsmaterial für das Ozon vorhanden ist. Bevorzugt handelt es sich bei dem Ventil um ein Magnetventil, welches mit der Steuervorrichtung der Sicherheitswerkbank geöffnet und geschlossen wird, um so eine vollautomatische Dekontamination durchführen zu können. Im gezeigten Fall ist das Ventil an der Frontseite der Sicherheitswerkbank angeordnet. Es ist jedoch ebenso gut möglich, dass Ventil 7 an anderer Stelle in der Sicherheitswerkbank vorzusehen, beispielsweise in einem Bereich des Gehäuses 3 unterhalb des Arbeitsraums 2. Auch die Anordnung des Ozongenerators 4 ist nicht auf den Raum oberhalb des Arbeitsraums 7 beschränkt. Ein alternativer Anbringungsort ist beispielsweise der hintere Lüftungskanal 10.

Zum Ablassen der Dekontaminationsluft aus der Sicherheitswerkbank 1 kann in der Abluftvorrichtung 6 ein für die Entlüftung vorgesehenes zusätzliches Gebläse vorgesehen sein. Dieses Gebläse fördert die Dekontaminationsluft aus dem Inneren der Sicherheitswerkbank 1 zunächst über den Filter 12 in die Vorrichtung 5 zum Abbau des Ozons. Innerhalb dieser Vorrichtung 5 ist ein Filter oder Katalysator, bevorzugt ein Platin-Iridium-Katalysator, angeordnet, welcher noch vorhandenes Ozon zu Sauerstoff abbaut. Die aus der Abluftvorrichtung 6 aus der Sicherheitswerkbank 1 austretende Luft ist für die Umgebung vollkommen unschädlich. Es verbleiben auch keine Reste von Desinfektionsmittel im Inneren der Sicherheitswerkbank, die im Anschluss an die Dekontaminierung bearbeitete Proben schädigen könnten.

## Patentansprüche

1. Sicherheitswerkbank (1) mit einem einen Arbeitsraum (2) umschließenden Gehäuse (3) und einem Lüftungssystem zur Belüftung des Arbeitsraums (2),
**dadurch gekennzeichnet,**
**dass** sie einen Ozongenerator (4) zur Einspeisung von Ozon in das Lüftungssystem umfasst.

2. Sicherheitswerkbank gemäß Anspruch 1,
**dadurch gekennzeichnet,**
**dass** der Ozongenerator (4) einen UV-Strahler oder ein Mittel zur elektrischen Ozonerzeugung umfasst.

3. Sicherheitswerkbank gemäß Anspruch 1 oder 2,
**dadurch gekennzeichnet,**
**dass** der Ozongenerator (4) im Bereich eines Lüftungskanals des Lüftungssystems außerhalb des Arbeitsraums (2) angeordnet ist.

4. Sicherheitswerkbank gemäß einem der Ansprüche 1 bis 3,
**dadurch gekennzeichnet,**
**dass** sie eine Vorrichtung (5) zum Abbau des Ozons umfasst.

5. Sicherheitswerkbank gemäß Anspruch 4,
**dadurch gekennzeichnet,**
**dass** die Vorrichtung (5) zum Abbau des Ozons in die Abluftvorrichtung (6) der Sicherheitswerkbank (1) integriert ist.

6. Sicherheitswerkbank gemäß Anspruch 4 oder 5,
**dadurch gekennzeichnet,**
**dass** die Vorrichtung (5) zum Ozonabbau einen Filter oder Katalysator, insbesondere einen Platin-lridium-Katalysator, umfasst.

7. Sicherheitswerkbank gemäß einem der Ansprüche 1 bis 6,
**dadurch gekennzeichnet,**
**dass** sie ein Ventil (7) zur Zufuhr von Umgebungsluft in das Belüftungssystem umfasst.

8. Sicherheitswerkbank gemäß einem der Ansprüche 1 bis 7,
**dadurch gekennzeichnet,**
**dass** sie eine aerosoldicht verschließbare Frontscheibe (8) zum Verschließen der Arbeitsöffnung des Arbeitsraums (2) aufweist.

9. Sicherheitswerkbank gemäß einem der Ansprüche 1 bis 8,
**dadurch gekennzeichnet,**
**dass** sie eine Steuervorrichtung zur mindestens teilweise automatischen Steuerung des Ozongenerators (4) und des Lüftungssystems aufweist.

10. Sicherheitswerkbank gemäß Anspruch 9,
**dadurch gekennzeichnet,**
**dass** die Steuervorrichtung Mittel zum Feststellen und Auswerten von Geräteparametern und/oder Mittel zum Anzeigen von Störungen umfasst.

11. Verfahren zur Dekontamination einer Sicherheitswerkbank (1), die einen vom einem Gehäuse (3) umschlossenen Arbeitsraum (2) und ein Lüftungssystem zur Belüftung des Arbeitsraums (2) umfasst,
**dadurch gekennzeichnet,**
**dass** in das Lüftungssystem von einem Ozongenerator (4) erzeugtes Ozon eingespeist und mittels des Lüftungssystems durch den Arbeitsraum (2) geleitet wird.

12. Verfahren gemäß Anspruch 11,
**dadurch gekennzeichnet,**
**dass** Erzeugung und Durchleitung des Ozons mehrere Stunden lang erfolgen.

13. Verfahren gemäß Anspruch 11 oder 12,
**dadurch gekennzeichnet,**
**dass** das Ozon nach Beendigung der Dekontamination über eine Vorrichtung (5) zum Abbau des Ozons geleitet wird.

14. Verfahren gemäß einem der Ansprüche 11 bis 13,
**dadurch gekennzeichnet,**
**dass** die Sicherheitswerkbank (1) vor Beginn der Ozonerzeugung gegen die Umgebung aerosoldicht abgeschlossen wird.

15. Verfahren gemäß einem der Ansprüche 1 bis 14,
**dadurch gekennzeichnet,**
**dass** die Dekontamination mit Ozon von einer Steuervorrichtung zumindest teilweise automatisch gesteuert wird.

16. Verfahren gemäß Anspruch 15,
**dadurch gekennzeichnet,**
**dass** die Sicherheitswerkbank (1) Mittel zum Feststellen und Auswerten von Geräteparametern und Mittel zum Anzeigen von Störungen umfasst und dass die Ozonerzeugung bei Feststellung einer Störung abgebrochen wird.

17. Verfahren gemäß einem der Ansprüche 1 bis 16,
**dadurch gekennzeichnet,**
**dass** der Arbeitsraum (2) vor und/oder nach der Dekontamination mit Ozon mit einem flüssigen Desinfektionsmittel, insbesondere einem Sprühdesinfektionsmittel, gereinigt wird.

18. Verwendung von Ozon zur Dekontamination einer Sicherheitswerkbank (1).
